**Europäisches Patentamt**

**European Patent Office**

**Office européen des brevets**

(19)

(11) Veröffentlichungsnummer : **0 490 053 B1**

(12) # EUROPÄISCHE PATENTSCHRIFT

(45) Veröffentlichungstag der Patentschrift :
**03.08.94 Patentblatt 94/31**

(51) Int. Cl.⁵ : **A61K 7/08, A61K 7/00**

(21) Anmeldenummer : **91117543.8**

(22) Anmeldetag : **15.10.91**

(54) **Haarkur in Form einer Mikroemulsion.**

(30) Priorität : **07.12.90 DE 4039063**

(43) Veröffentlichungstag der Anmeldung :
**17.06.92 Patentblatt 92/25**

(45) Bekanntmachung des Hinweises auf die
Patenterteilung :
**03.08.94 Patentblatt 94/31**

(84) Benannte Vertragsstaaten :
**DE ES FR GB IT**

(56) Entgegenhaltungen :
**EP-A- 0 278 660**
**DE-A- 1 467 825**
**FR-A- 2 345 997**

(56) Entgegenhaltungen :
**Römpps Chemie-Lexikon, Franckhsche Verlagsbuchhandlung, 8.Auflage, 1987, Seiten
3363 und 2878**
**Taschenbuch für Chemiker und Physiker,
D'Ans Lax, 3.Auflage, Springer Verlag 1964,
Seite 2-743**

(73) Patentinhaber : **Wella Aktiengesellschaft**
**Berliner Allee 65**
**D-64295 Darmstadt (DE)**

(72) Erfinder : **Schröder, Friedel**
**Viktoriaplatz 1**
**W-6100 Darmstadt (DE)**
Erfinder : **Lang, Günther, Dr.**
**Auf der Roten Erde 10 B**
**W-6107 Reinheim 5 (DE)**

**Beschreibung**

Gegenstand der Erfindung ist ein Haarkurmittel in Form einer Mikroemulsion, das mindestens ein Öl, mindestens ein nichtionisches Tensid mit einem HLB-Wert von 5 bis 12, mindestens ein quaternäres Tensid und Wasser enthält und eine hochviskose gelartige Konsistenz aufweist.

Die Haare werden durch Einwirkungen verschiedener Art in ihren physikalischen, chemischen und morphologischen Eigenschaften negativ beeinflußt. So wird durch kosmetische Behandlungen, wie wiederholtes Bleichen, Dauerwellen und Färben, aber auch durch häufiges Waschen der Haare mit entfettenden Tensiden sowie durch Klimaeinflüsse wie Feuchte- und Temperaturunterschiede oder die intensive Einwirkung von Sonnenlicht die Haarstruktur geschädigt. Das Haar wird spröde und verliert seinen Glanz. Das derart geschädigte Haar lädt sich beim Kämmen und Bürsten elektrostatisch auf, und die aufgerauhte Haaroberfläche führt durch die Bildung von Verfilzungen und Verknotungen zu einer schlechten Kämmbarkeit und Entwirrbarkeit des Haares. Haarkurmittel mit einer pflegenden und kämmbarkeitsverbessernden Wirkung haben daher erhebliche Bedeutung erlangt.

Haarkurmittel dienen der Verbesserung des Haarzustandes und liegen üblicherweise in Form von Emulsionen oder Suspensionen vor, die Fettalkohole, Wachse und Öle sowie anionische und kationische Tenside enthalten. Haarkurmittel sind üblicherweise trübe flüssige oder hochviskose Zubereitungen.

Es hat in der Vergangenheit nicht an Versuchen gefehlt, klare Kurpackungen oder Haarspülungen herzustellen.

So sind aus der FR-A- 2 345 997 dünnflüssige klare Haar spülungen bekannt, deren Transparenz durch den Zusatz von 5 bis 30 Gewichtsprozent eines alkoholischen Lösungsmittels bewirkt wird.

Aus der DE-A- 1 467 825 sind klare Frisiermittel in Form viskoser Mineralöl-Wasser-Gele bekannt.

Um hochviskose Haarkurmittel zur Verfügung zu stellen, wurden beispielsweise wäßrige Lösungen kationischer Tenside oder kationischer Polymere mittels nichtionischer Polymere verdickt. Derartige Zusammensetzungen erreichen jedoch bis heute nicht die konditionierende Wirkung kationischer Emulsionen.

Im Gegensatz zu den üblicherweise verwendeten zwei- oder mehrphasigen Haarkurmitteln, die das Risiko eines Absetzens, beziehungsweise Auftrennens der Phasen beinhalten, sind Mikroemulsionen klare, thermodynamisch stabile, quasi - einphasige Systeme.

Aus der EP-A- 0 278 660 sind klare homogene Mikroemulsionen zur topischen Behandlung von Haut und Haaren bekannt, die mindestens 20 Gewichtsprozent einer hydrophoben Ölphase, 0,01 bis 20 Gewichtsprozent eines quaternären Ammoniumtensids und 0,01 bis 20 Gewichtsprozent einer hydrophilen Phase, die zum Beispiel aus Wasser bestehen kann, sowie eine ausreichende Menge eines Cotensids enthalten. Die in den Ausführungsbeispielen der EP-A- 0 278 660 beschriebenen Mikroemulsionen betreffen keine Haarkurmittel und weisen zudem einen relativ niedrigen Wassergehalt (maximal 6 Gewichtsprozent) und einen hohen Ölanteil auf. Die dort beschriebenen Mikroemulsionen sind daher als Haarkurmittel nur wenig geeignet, da sie die Haare stark mit Öl belasten, was ein schlechtes Ausspülverhalten des Mittels und ein fettiges und strähniges Aussehen der Haare zur Folge hat.

Die in der EP-A- 0 278 660 beschriebenen Mikroemulsionen sind zudem dünnflüssig. Diese dünnflüssige Konsistenz ist für Haarkurmittel ungeeignet, da diese dünnflüssigen Mittel während der für die Wirkung des Haarkurmittels wesentlichen Einwirkungszeit bereits wieder vom Haar abtropfen.

Es bestand daher die Aufgabe, ein Haarkurmittel in Form einer Mikroemulsion zur Verfügung zu stellen, das die Haare nicht durch einen hohen Ölgehalt mit Öl belastet und eine hochviskose, gelartige Konsistenz aufweist, die das Abtropfen des Mittels während der Einwirkungszeit verhindert. Weiterhin soll dieses Mittel eine gute Konditionierung des Haares gewährleisten und die Naß- und Trockenkämmbarkeit sowie den Griff und den Glanz des trockenen Haares verbessern.

Es wurde nun gefunden, daß ein Haarkurmittel in Form einer Mikroemulsion, welches einen Gehalt an
a) 5 bis 20 Gewichtsprozent eines nicht-ionischen Tensids mit einem HLB-Wert von 5 bis 12 oder eines Gemisches dieser Tenside, wobei der HLB-Wert des Tensidgemisches 6 bis 10 beträgt,
b) 5 bis 20 Gewichtsprozent mindestens eines Öls,
c) 0,5 bis 10 Gewichtsprozent mindestens eines kationischen Tensids
und
d) 50 bis 89,5 Gewichtsprozent Wasser
aufweist und keine nicht-ionischen Tenside mit einem HLB-Wert größer als 12 enthält, die gestellte Aufgabe in hervorragender Weise erfüllt.

Das erfindungsgemäße Mittel läßt sich sehr gut ins Haar einarbeiten, tropft aufgrund seiner hochviskosen Konsistenz nicht ab, und bewirkt eine ausgezeichnete Verbesserung der Naß- und Trockenkämmbarkeit des Haares sowie eine verbesserten Griff insbesondere des geschädigten Haares. Darüberhinaus ist ein hervorragender Glanz des Haares nach der Behandlung mit dem erfindungsgemäßen Mittel festzustellen. Das erfindungsgemäße Haarkurmittel weist zudem eine optisch ansprechende klare homogene, gelartig hochviskose Konsistenz auf.

Im Gegensatz zu den aus der EP-A- 0 278 660 bekannten, beispielsweise in den Beispielen 5 und 8 der EP-A- 0 276 660 beschriebenen, dünnflüssigen Mikroemulsionen, die das Fließverhalten Newtonscher Flüssigkeiten (Haake Rotationsviskosimeter RV 12, Meßteil 500, Meßsystem NV, 20° Celsius) zeigen, weist das gelartig hochviskose erfindungsgemäße Mittel ein Fließverhalten wie plastische Körper auf. Die für das Fließverhalten plastischer Körper typische Fließgrenze liegt beispielsweise für das Haarkurmittel gemäß Beispiel 15 dieser Anmeldung bei 146 Pascal (Haake Rotationsviskosimeter RV 12, Meßteil 500, Meßsystem PKV-0.5, 20° Celsius).

Unter Haarkurmitteln werden in der vorliegenden Anmeldung sowohl Mittel verstanden, die zur Entfaltung ihrer pflegenden Wirkung nur kurz, beispielsweise 2 bis 5 Minuten lang auf das Haar einwirken, sogenannte Spülungen oder Konditioner, als auch Mittel die über einen längeren Zeitraum, beispielsweise 5 bis 30 Minuten lang, auf das Haar einwirken und üblicherweise als Haarkuren oder Kurpackungen bezeichnet werden.

Das hier beschriebene Haarkurmittel soll nur nicht-ionische Tenside mit einem HLB-Wert von 5 bis 12, bevorzugt jedoch mit einem HLB-Wert von 6 bis 10, enthalten. Enthält das erfindungsgemäße Mittel ein Gemisch mehrerer nicht-ionischer Tenside, die einen HLB-Wert von 5 bis 12, bevorzugt jedoch einen HLB-Wert von 6 bis 10, aufweisen, so soll der HLB-Wert des Tensidgemisches 6 bis 10 betragen. Unter dem HLB-Wert wird in dieser Anmeldung der Wert für die Hydrophile-Lipophile Balance verstanden, wie sie in Lehrbüchern, zum Beispiel in G. Nowak, Die kosmetischen Präparate, Band 2, 3. Auflage (1984), Seiten 174 bis 177, definiert wird.

Von den für das erfindungsgemäße Haarkurmittel geeigneten nicht-ionischen Tensiden seien beispielsweise die folgenden genannt: mit 1 bis 6, bevorzugt jedoch mit 2 bis 5, Ethylenoxideinheiten ethoxylierte $C_{12}$- bis $C_{18}$-Fettalkohole, zum Beispiel mit 2 bis 5 Mol Ethylenoxid pro Mol Fettalkohol ethoxylierter Lauryl-, Cetyl-, Oleyl- oder Stearylalkohol; Polyglycerylether von gesättigten und ungesättigten $C_{12}$- bis $C_{18}$-Fettalkoholen mit 1 bis 5, bevorzugt jedoch 1 bis 3, Glyceryleinheiten im Molekül; Glyceride von $C_{12}$- bis $C_{18}$-Fettsäuren mit 1 bis 5, bevorzugt jedoch 1 bis 3, Glyceryleinheiten im Molekül, wie beispielsweise Glycerinmonolaurat, Diglycerinmonolaurat, Triglycerinmonolaurat, Glycerinmonomyristat oder Triglycerinmonoisostearat; mit 1 bis 6 Ethylenoxideinheiten ethoxylierte Sorbitanfettsäureester von $C_{12}$- bis $C_{18}$-Fettsäuren und $C_{12}$- bis $C_{18}$-Fettsäureglycoside mit 1 bis 3 Zuckereinheiten, beispielsweise Glucoseeinheiten, im Molekül.

Das nicht-ionische Tensid oder das Tensidgemisch ist in dem erfindungsgemäßen Haarkurmittel in einer Menge von 5 bis 20 Gewichtsprozent, vorzugsweise 8 bis 15 Gewichtsprozent, enthalten.

Als Öl können in dem erfindungsgemäßen Mittel natürliche oder synthetische Öle oder ein Gemisch aus natürlichen und synthetischen Ölen verwendet werden. Von den für die Verwendung in dem erfindungsgemäßen Haarkurmittel geeigneten natürlichen oder synthetischen Ölen seien beispielsweise die folgenden erwähnt: Paraffinöle; geradkettige Fettsäureester, wie zum Beispiel Myristylmyristinat, Laurinsäurehexylester oder Ölsäureoleylester; verzweigte Fettsäureester wie beispielsweise Isononansäurecetyl- oder -stearylester; Isooctylfettsäureester, wie zum Beispiel Isooctylstearat; Silikonöle wie beispielsweise Octamethyltetracyclosiloxan; Squalan und pflanzliche Öle wie zum Beispiel Jojobaöl.

Das erfindungsgemäße Haarkurmittel enthält 5 bis 20 Gewichtsprozent, bevorzugt 11 bis 19 Gewichtsprozent, mindestens eines Öls.

Als kationische Tenside können alle für die Verwendung in kosmetischen Mitteln geeigneten kationischen Tenside, vorzugsweise jedoch quaternäre Alkylammoniumverbindungen, verwendet werden. Als geeignete Tenside seien beispielsweise die folgenden genannt: Benzyldialkylammoniumchloride oder -bromide, wie beispielsweise Benzyldimethylstearylammoniumchlorid; Alkyltrimethylammoniumsalze, beispielsweise Cetyltrimethylammoniumchlorid oder -bromid; Alkyldimethylhydroxyethylammoniumchloride oder -bromide; Dialkyldimethylammoniumchloride oder -bromide; Alkylamidethyltrimethylammoniumethersulfate; Alkylpyridiumsalze, beispielsweise Lauryl- oder Cetylpyridiniumchlorid; Imidazolinderivate und Verbindungen mit kationischem Charakter wie Aminoxide, beispielsweise Alkyldimethylaminoxide oder Alkylaminoethyldimethylaminoxide.

Das erfindungsgemäße Haarkurmittel enthält 0,5 bis 10 Gewichtsprozent, vorzugsweise 1,5 bis 6 Gewichtsprozent, mindestens eines kationischen Tensids.

Der Wassergehalt des erfindungsgemäßen Haarkurmittels beträgt 50 bis 89,5 Gewichtsprozent, vorzugsweise 60 bis 80 Gewichtsprozent.

Selbstverständlich können auf der Basis der erfindungsgemäßen klaren Haarkurmittel auch getrübte oder perlglanzgetrübte Haarkurmittel hergestellt werden, indem dem erfindungsgemäßen Haarkurmittel zum Beispiel Trübungsmittel in einer Menge von 0,5 bis 5 Gewichtsprozent, beispielsweise Ethylglykoldistearat, oder Perlglanzmittel in einer Menge von 1,0 bis 10,0 Gewichtsprozent, wie beispielsweise ein Gemisch aus Fettsäuremonoalkylolamid und Ethylenglykoldistearat, zugesetzt werden.

Das an sich klare Aussehen der hier beschriebenen Haarkurmittel kann auch durch Feststoffe wie Metallflitter, feinverteiltes Siliciumdioxid oder durch den Zusatz von wasserunlöslichen Antischuppenwirkstoffen, wie beispielsweise Zink-Omadine, verändert werden.

Selbstverständlich kann das erfindungsgemäße Haarkurmittel neben den genannten Bestandteilen noch für Haarbehandlungsmittel übliche weitere Zusätze, beispielsweise Parfümöle in einer Menge von 0,5 bis 5,0 Gewichtsprozent; Verdickungsmittel, beispielsweise Kokosfettsäurediethanolamid, in einer Menge von 0,5 bis 10,0 Gewichtsprozent; Verdünnungsmittel, zum Beispiel 1,2 Propylenglykol oder ethoxyliertes Sorbitanmonolaurat, in einer Menge von 0,5 bis 5,0 Gewichtsprozent; Puffersubstanzen, beispielsweise Natriumcitrat oder Natriumphosphat, in einer Menge von 0,1 bis 1,0 Gewichtsprozent; haarkonditionierende Zusätze, zum Beispiel Fettsäuren, Fettalkohole, Protein-Partialhydrolysate, modifizierte natürliche oder synthetische, vorzugsweise kationische, Polymere, wie Cellulosederivate, kationische Cellulosederivate, Chitosan und kationische Chitosanderivate; Pflegestoffe, beispielsweise Lanolinderivate, Cholesterin und Pantothensäure in einer Menge von 0,1 bis 10 Gewichtsprozent; außerdem physiologisch verträgliche anorganische Salze, zum Beispiel Natriumchlorid; ferner Feuchthaltemittel; Lichtschutzmittel; bakterizide und fungizide Stoffe; Anfärbefarbstoffe; Antioxidantien zum Beispiel Ascorbinsäure, und reduzierende Verbindungen, beispielsweise Glyoxylsäure; Komplexbildner; Antifett- und Antischuppenwirkstoffe enthalten.

In einer besonderen Ausführungsform des erfindungsgemäßen Haarkurmittels enthält dieses zur gleichzeitigen Tönung des Haares 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C.I. 14 805), Anthrachinonfarbstoffe, zum Beispiel Disperse Violet 4 (C.I. 61 105) und Triphenylmethanfarbstoffe, zum Beispiel Basic Violet 1 (C.I. 42 535), wobei die Farbstoffe je nach Art ihrer Substituenten sauren, nichtionogenen oder basischen Charakter haben können.

Das erfindungsgemäße Haarkurmittel kann hergestellt werden, indem zunächst die 60 bis 70 ° Celsius warme wäßrige Lösung des kationischen Tensids in eine Lösung des nichtionischen Tensids im Öl gleicher Temperatur unter kräftigem Rühren eingearbeitet wird. Beim Abkühlen erstarrt das erfindungsgemäße Haarkurmittel zu einem klaren Gel. Bei geeigneten Komponenten ist es auch möglich, ohne vorherige Erwärmung die flüssige Mischung aus Ölphase und nichtionischem Tensid in einer geeigneten Mischvorrichtung unter Vermeidung von Lufteinschlüssen mit der Lösung des kationischen Tensids in Wasser zu homogenisieren.

Das erfindungsgemäße Haarkurmittel wird, üblicherweise nach der Haarwäsche, in dem handtuchtrockenen Haar je nach Haarfülle in einer Menge von etwa 10 bis 30 g verteilt. Nach einer Einwirkungszeit von 1 bis 30 Minuten, vorzugsweise 3 bis 15 Minuten, wird das Haar mit Wasser ausgespült und sodann getrocknet.

Die Einwirkungszeit des erfindungsgemäßen Mittels hängt innerhalb der gegebenen zeitlichen Grenzen von dem Verwendungszweck des Mittels ab. Handelt es sich um eine Spülung, so beläßt man sie 1 bis 5 Minuten auf dem Haar, während bei einer Haarkur eine Einwirkungsdauer von 3 bis 15 Minuten üblich ist und bei einer Haarkur mit gleichzeitiger Tönung des Haares die Einwirkungszeit 10 bis 30 Minuten beträgt.

Das erfindungsgemäße Haarkurmittel läßt sich gut in dem zuvor gewaschenen, handtuchtrockenen Haar verteilen und ist nach der Einwirkungszeit problemlos wieder aus dem Haar zu spülen.

Die mit dem erfindungsgemäßen Haarkurmittel behandelten Haare zeigen eine hervorragende Naß- und Trockenkämmbarkeit, einen angenehmen Griff und einen ansprechenden Glanz im getrockneten Zustand.

Die nachstehenden Beispiele sollen den Gegenstand der Erfindung näher erläutern.

**Beispiele**

**Beispiel 1:**     **Haarkurmittel**

| | |
|---|---|
| 11,25 g | Paraffinöl |
| 5,50 g | Tetraoxyethylenlaurylether |
| 4,50 g | Glycerinmonolaurat |
| 1,88 g | Cetyltrimethylammoniumchlorid |
| 76,87 g | Wasser, vollentsalzt |
| 100,00 g | |

Beispiel 2:          Haarkurmittel

8,40 g               Tetraoxyethylenlaurylether
7,50 g               Ölsäureoleylester
6,00 g               Paraffinöl
3,60 g               Glycerinmonolaurat
2,25 g               Cetyltrimethylammoniumchlorid
72,25 g              Wasser, vollentsalzt
100,00 g


Beispiele 3:         Haarkurmittel

13,50 g              Gemisch des Cetyl- und Stearyl-
                     esters der Isononansäure
7,80 g               Tetraoxyethylenlaurylether
4,20 g               Glycerinmonolaurat
2,25 g               Cetyltrimethylammoniumchlorid
72,25 g              Wasser, vollentsalzt
100,00 g


Beispiele 4:         Haarkurmittel

13,50 g              Octamethylcyclotetrasiloxan
12,00 g              Trioxyethylenlaurylether
2,25 g               Cetyltrimethylammoniumchlorid
72,25 g              Wasser, vollentsalzt
100,00 g


Beispiel 5:          Haarkurmittel

13,50 g              Isooctylstearat
8,40 g               Tetraoxyethylenlaurylether
3,60 g               Glycerinmonolaurat
2,25 g               Cetyltrimethylammoniumchlorid
72,25 g              Wasser, vollentsalzt
100,00 g

**Bespiel 6:**      **Haarkurmittel**

| 12,25 g | Squalan |
|---|---|
| 4,50 g | Glycerinmonolaurat |
| 3,50 g | Tetraoxyethylenlaurylether |
| 1,88 g | Cetyltrimethylammoniumchlorid |
| 77,87 g | Wasser, vollentsalzt |
| 100,00 g | |

**Beispiel 7:**      **Haarkurmittel**

| 11,25 g | Laurinsäurehexylester |
|---|---|
| 7,00 g | Tetraoxyethylenlaurylether |
| 3,00 g | Glycerinmonolaurat |
| 1,88 g | Cetyltrimethylammoniumchlorid |
| 76,87 g | Wasser, vollentsalzt |
| 100,00 g | |

**Beispiel 8:**      **Haarkurmittel**

| 9,00 g | Paraffinöl |
|---|---|
| 9,00 g | Tetraoxyethylenlaurylether |
| 4,50 g | 9-Octadecenyl-13-docosenoat |
| 3,00 g | Glycerinmonolaurat |
| 2,25 g | Cetyltrimethylammoniumchlorid |
| 72,25 g | Wasser, vollentsalzt |
| 100,00 g | |

**Beispiel 9:**      **Haarkurmittel**

| 13,50 g | Paraffinöl |
|---|---|
| 6,05 g | Tetraoxyethylenlaurylether |
| 4,95 g | Glycerinmonolaurat |
| 3,78 g | Cetyldimethylbenzylammoniumchlorid |
| 71,72 g | Wasser, vollentsalzt |
| 100,00 g | |

**Beispiel 10:**     **Haarkurmittel**

11,25 g     Paraffinöl
5,50 g     Tetraoxyethylenlaurylether
4,50 g     Glycerinmonolaurat

2,48 g     Cetyldimethyl-2-hydroxyethyl-
            ammoniumdihydrogenphosphat
76,27 g     Wasser, vollentsalzt
100,00 g


**Beispiel 11:**     **Haarkurmittel**

13,50 g     Paraffinöl
8,00 g     Diglycerinmonolaurat
4,00 g     Glycerinmonolaurat
2,25 g     Cetyltrimethylammoniumchlorid
72,25 g     Wasser, vollentsalzt
100,00 g


**Beispiel 12:**     **Haarkurmittel**

13,50 g     Paraffinöl
6,00 g     Glycerinmonolaurat
6,00 g     Triglycerinmonoisostearat
2,25 g     Cetyltrimethylammoniumchlorid
72,25 g     Wasser, vollentsalzt
100,00 g

7

Beispiel 13:          Haarkurmittel

| | |
|---|---|
| 13,50 g | Paraffinöl |
| 7,00 g | Glycerinmonomyristat |
| 5,00 g | Triglycerinmonolaurat |
| 2,25 g | Cetyltrimethylammoniumchlorid |
| 72,25 g | Wasser, vollentsalzt |
| 100,00 g | |


Beispiel 14:          Haarkurmittel

| | |
|---|---|
| 15,00 g | Paraffinöl |
| 7,50 g | Stearatester des Sorbitols und des Sorbitolanhydrids, mit 4 Mol Ethylenoxid ethoxyliert |
| 7,00 g | Dioxyethylenlaurylether |
| 2,85 g | Cetyltrimethylammoniumchlorid |
| 67,65 g | Wasser, vollentsalzt |
| 100,00 g | |


Beispiel 15:          Haarkurmittel

| | |
|---|---|
| 11,250 g | Paraffinöl |
| 5,500 g | Tetraoxyethylenlaurylether |
| 4,500 g | Glycerinmonolaurat |
| 1,875 g | Trimethylhexadecylammoniumchlorid |
| 76,875 g | Wasser, vollentsalzt |
| 100,000 g | |

**Beispiel 16:**     **Haarkurmittel**

| | | |
|---|---|---|
| 15,75 | g | Paraffinöl |
| 12,75 | g | Pentaoxyethylenoleylether |
| 1,75 | g | Trimethylhexadecylammoniumchlorid |
| 1,50 | g | Tetraoxyethylenlaurylether |
| 1,00 | g | Parfümöl |
| 0,20 | g | Zitronensäure |
| 67,05 | g | Wasser, vollentsalzt |
| 100,00 | g | |

Jeweils 20 g des Haarkurmittels gemäß den Beispielen 1 bis 16 werden in dem zuvor gewaschenen, handtuchtrockenen Haar verteilt. Nach einer Einwirkungszeit von 3 bis 15 Minuten wird das Haar mit Wasser gespült und sodann getrocknet. Als Ergebniss wird eine ausgezeichnete Kämmbarkeit, ein sehr guter Griff sowie ein ansprechender Glanz des Haares erhalten.

Sämtliche in der Anmeldung angegebenen Prozentzahlen stellen Gewichtsprozente dar.

**Patentansprüche**

1. Haarkurmittel in Form einer Mikroemulsion, dadurch gekennzeichnet, daß es einen Gehalt an
   a) 5 bis 20 Gewichtsprozent eines nicht-ionischen Tensids mit einem HLB-Wert von 5 bis 12 oder eines Gemisches dieser Tenside, wobei der HLB-Wert des Tensidgemisches 6 bis 10 beträgt,
   b) 5 bis 20 Gewichtsprozent mindestens eines Öls,
   c) 0,5 bis 10 Gewichtsprozent mindestens eines kationischen Tensids und
   d) 50 bis 89,5 Gewichtsprozent Wasser
   aufweist und keine nicht-ionischen Tenside mit einem HLB-Wert größer als 12 enthält.

2. Mittel nach Anspruch 1, dadurch gekennzeichnet, daß das nicht-ionische Tensid oder das Tensidgemisch in einer Menge von 8 bis 15 Gewichtsprozent enthalten ist.

3. Mittel nach Anspruch 1 oder 2, dadurch gekennzeichnet, daß das nicht-ionische Tensid ausgewählt ist aus: mit 1 bis 6 Ethylenoxideinheiten ethoxylierten $C_{12}$- bis $C_{18}$-Fettalkoholen, Polyglycerylethern von gesättigten oder ungesättigten $C_{12}$-bis $C_{18}$-Fettalkoholen mit 1 bis 5 Glyceryleinheiten im Molekül; Glyceriden von $C_{12}$ - bis $C_{18}$-Fettsäuren mit 1 bis 5 Glyceryleinheiten im Molekül; mit 1 bis 6 Ethylenoxideinheiten ethoxylierte Sorbitanfettsäureestern von $C_{12}$ - bis $C_{18}$-Fettsäuren und $C_{12}$ -bis $C_{18}$-Fettsäureglycosiden mit 1 bis 3 Zuckereinheiten im Molekül.

4. Mittel nach einem der Ansprüche 1 bis 3, dadurch gekennzeichnet, daß als Öl ein natürliches oder synthetisches Öl oder ein Gemisch aus natürlichen oder synthetischen Ölen verwendet wird.

5. Mittel nach einem der Ansprüche 1 bis 4, dadurch gekennzeichnet, daß das Öl ausgewählt ist aus: Paraffinölen, geradkettigen und verzweigten Fettsäureestern, Isooctylfettsäureestern, Silikonölen, Squalan und pflanzlichen Ölen.

6. Mittel nach einem der Ansprüche 1 bis 5, dadurch gekennzeichnet, daß es 11 bis 19 Gewichtsprozent mindestens eines Öls enthält.

7. Mittel nach einem der Ansprüche 1 bis 6, dadurch gekennzeichnet, daß das kationische Tensid ausgewählt ist aus Benzyldialkylammoniumchloriden oder -bromiden, Alkyltrimethylammoniumsalzen, Alkyldimetylhydroxyethylammoniumchloriden oder -bromiden, Dialkyldimethylammoniumchloriden oder -bromiden, Alkylamidetyltrimethylammoniumethersulfaten, Alkylpyridiumsalzen, Imidazolinderivaten und Aminoxiden.

8. Mittel nach einem der Ansprüche 1 bis 7, dadurch gekennzeichnet, daß es 1,5 bis 6 Gewichtsprozent mindestens eines kationischen Tensids enthält.

9. Mittel nach einem der Ansprüche 1 bis 8, dadurch gekennzeichnet, daß es 60 bis 80 Gewichtsprozent Wasser enthält.

10. Mittel nach einem der Ansprüche 1 bis 9, dadurch gekennzeichnet, daß es zur gleichzeitigen Tönung des Haares 0,05 bis 2,0 Gewichtsprozent mindestens eines direkt auf das Haar aufziehenden Haarfarbstoffs enthält.

**Claims**

1. Hair treatment agent in the form of a microemulsion, characterised in that it contains:
   a) 5 to 20 weight % of a non-ionic surfactant with an HLB value of 5 to 12, or a mixture of these surfactants, the HLB value of the surfactant mixture being 6 to 10;
   b) 5 to 20 weight % of at least one oil;
   c) 0.5 to 10 weight % of at least one cationic surfactant; and
   d) 50 to 89.5 weight % of water and does not contain any non-ionic surfactants with an HLB value of more than 12.

2. Agent according to Claim 1, characterised in that the non-ionic surfactant or the surfactant mixture is present in an amount of from 8 to 15 weight %.

3. Agent according to Claim 1 or 2, characterised in that the non-ionic surfactant is selected from: $C_{12}$ to $C_{18}$ fatty alcohols oxyethylated with 1 to 6 ethylene oxide units, polyglyceryl ethers of saturated or unsaturated $C_{12}$ to $C_{18}$ fatty alcohols with 1 to 5 glyceryl units in the molecule; glycerides of $C_{12}$ to $C_{18}$ fatty acids with 1 to 5 glyceryl units in the molecule; $C_{12}$ to $C_{18}$ fatty acid sorbitan fatty acid esters oxyethylated with 1 to 6 ethylene oxide units, and $C_{12}$ to $C_{18}$ fatty acid glycosides with 1 to 3 sugar units in the molecule.

4. Agent according to any one of Claims 1 to 3, characterised in that a natural or synthetic oil or a mixture of natural or synthetic oils is used as the oil.

5. Agent according to any one of Claims 1 to 4, characterised in that the oil is selected from: paraffin oils, straight-chain and branched fatty acid esters, isooctyl fatty acid esters, silicone oils, squalene and vegetable oils.

6. Agent according to any one of Claims 1 to 5, characterised in that it contains 11 to 19 weight % of at least one oil.

7. Agent according to any one of Claims 1 to 6, characterised in that the cationic surfactant is selected from benzyldialkylammonium chlorides or bromides, alkyltrimethylammonium salts, alkyldimethylhydroxyethylammonium chlorides or bromides, dialkyldimethylammonium chlorides or bromides, alkylamidetyltrimethylammonium ether sulphates, alkylpyridium salts, imidazoline derivatives and aminooxides.

8. Agent according to any one of Claims 1 to 7, characterised in that it contains from 1.5 to 6 weight % of at least one cationic surfactant.

9. Agent according to any one of Claims 1 to 8, characterised in that it contains from 60 to 80 weight % of water.

10. Agent according to any one of Claims 1 to 9, characterised in that it contains from 0.05 to 2.0 weight % of at least one direct-acting hair dye for simultaneously tinting the hair.

**Revendications**

1. Agent de soins capillaires sous forme d'une micro-émulsion, caractérisé en ce qu'il contient une teneur en:

   a) 5 à 20% en poids d'un agent tensio-actif non ionique présentant une valeur HLB de 5 à 12, ou un mélange de ces agents tensio-actifs, la valeur HLB du mélange étant de 6 à 10,

   b) 5 à 20% en poids d'au moins une huile;

   c) 0,5 à 10% en poids d'au moins un agent tensio-actif cationique; et

   d) 50 à 89,5% en poids d'eau,

   et aucun agent tensio-actif cationique dont la valeur HLB soit supérieure à 12.

2. Agent selon la revendication 1, caractérisé en ce qu'il contient l'agent tensioactif non ionique ou le mélange d'agent tensio-actif à raison de 8 à 15% en poids.

3. Agent selon la revendication 1 ou 2, caractérisé en ce que l'agent tensioactif non ionique est choisi parmi les alcools gras en $C_{12}$ à $C_{18}$ éthoxylés avec 1 à 6 motifs oxyde d'éthylène, les éthers de polyglycérides d'alcools gras en $C_{12}$ à $C_{18}$, saturés ou non saturés avec 1 à 5 motifs glycéryle dans la molécule; des glycérides d'acides gras en $C_{12}$ à $C_{18}$ avec 1 à 5 motifs glycéryle dans la molécule ; les esters d'acides gras en $C_{12}$ à $C_{18}$ du sorbitane éthoxylés avec 1 à 6 motifs oxyde d'éthylène et des glucosides d'acides gras en $C_{12}$ à $C_{18}$ avec 1 à 3 motifs sucre dans la molécule.

4. Agent selon l'une des revendications 1 à 3, caractérisé en ce que on utilise comme huile une huile naturelle ou synthétique ou un mélange d'huiles naturelles et synthétiques.

5. Agent selon l'une des revendications 1 à 4, caractérisé en ce que l'on choisit l'huile parmi les huiles de paraffine, les esters d'acides gras à chaîne droite ou ramifiée, les esters d'acides gras et d'isooctyle, les huiles de silicone, le squalane et les huiles végétales.

6. Agent selon une des revendications 1 à 5, caractérisé en ce qu'il contient 11 à 19% en poids d'au moins une huile.

7. Agent selon l'une des revendications 1 à 6, caractérisé en ce que l'agent tensio-actif cationique est choisi parmi les chlorures ou les bromures de benzyldialkylammonium, les sels d'alkyltriméthylammonium, les chlorures ou les bromures d'alkyldiméthylhydroxyéthylammonium, les chlorures ou les bromures de dialkyldiméthyl-ammonium, les éther-sulfates d'alkylamidoéthyltriméthyl-ammonium, les sels d'alkylpyridinium, les dérivés d'imidazoline, et les oxydes d'amines.

8. Agent selon l'une des revendications 1 à 7, caractérisé en ce qu'il contient 1,5 à 6% en poids d'au moins un agent tensio-actif cationique.

9. Agent selon l'une des revendications 1 à 8, caractérisé en ce qu'il contient 60 à 80% en poids d'eau.

10. Agent selon l'une des revendications 1 à 9, caractérisé en ce qu'il contient pour un nuançage simultané des cheveux 0,05 à 2,0% en poids d'un colorant pour cheveux se fixant directement sur ceux-ci.